# EUROPEAN PATENT SPECIFICATION

(11) **EP 1 740 141 B1**
(45) Date of publication and mention of the grant of the patent: **15.09.2010**
(21) Application number: 05713697.0
(22) Date of filing: 14.02.2005
(51) Int. Cl.: A61F 13/56, A61F 13/551, A61F 13/15

(54) **EASY OPEN DIAPER**
EINFACH ZU ÖFFNENDE WINDEL
COUCHE-CULOTTE FACILE A OUVRIR

(30) Priority: 29.04.2004 US 836489
(43) Date of publication of application: 10.01.2007
(73) Proprietor: KIMBERLY-CLARK WORLDWIDE, INC., Neenah, WI 54956 (US)
(72) Inventor: DATTA, Paul, J., Appleton, Wisconsin 54913 (US)
(74) Representative: Mabey, Katherine Frances
(86) International application number: PCT/US2005/004987
(87) International publication number: WO 2005/110321

(56) References cited:
- EP-A- 0 648 483
- EP-A- 1 166 735
- GB-A- 1 441 567
- US-A- 3 938 523
- US-A- 5 577 540
- US-A1- 2001 034 512

## Description

### Background

The present invention relates to methods of folding. More particularly, the present invention relates to methods of folding disposable absorbent articles. The present invention also relates to folded disposable absorbent articles that are easy to use.

Absorbent articles such as, for example, diapers and adult incontinence garments are generally available to users in packages which include multiple articles therein. The disposable absorbent articles contained in these packages are typically folded to allow for efficient manufacturing, as well as for efficient space utilization within the packages. These disposable absorbent articles are generally folded such that the side margins are folded toward longitudinal centerline and also folded in half before insertion into the package. The result of this method of folding is that the user is presented with a disposable absorbent article that is compact in size but must be unfolded one or more times before individual components are accessible. More specifically, the fasteners that are present on many disposable absorbent articles are folded within the disposable absorbent article. Therefore, the disposable absorbent article must be unfolded one or more times before the fasteners can be accessed. This multi step process to access the fasteners may be an issue when applying the disposable absorbent article on an active child, or applying the disposable absorbent article at night, in the dark.

As a result, there has remained a need for a method of folding a disposable absorbent article that provides a relatively compact article. Moreover, there has remained a need for a method of folding a disposable absorbent article that provides a folded article that is easy to unfold. The also has remained a need for folded disposable absorbent articles that are easy to unfold.

US 3,938,523 discloses a prefolded and packaged disposable diaper. GB 1441567 discloses fasteners for diapers. EP 1166735 discloses a disposable diaper.

### Summary

The present inventors undertook intensive research and development efforts concerning methods of folding a disposable absorbent article that provided a folded article that is easy to unfold. While conducting their research, the present inventors discovered unique methods of folding disposable absorbent articles that resulted in folded disposable articles that are easy to unfold. The present invention provides a method of folding a disposable absorbent article in accordance with claim 1.

The present invention also provides a disposable absorbent article in accordance with claim 5.

In embodiments, the present invention may provide a folded disposable absorbent article including a longitudinal centerline, a transverse center line, side margins, opposing transverse end edges, a front region, a rear region and a crotch region. The crotch region extends between and connects the front region and the rear region. The folded disposable absorbent article includes a pair of ears associated with the rear region. The disposable absorbent article includes at least a first, second and third longitudinally extending folds in each side margin and one transversely extending fold. Where at least a portion of each of the ears extends beyond the first, second and third longitudinally extending folds in each side margin. The portions of the ears are colored to connote the portions location. The portions extend at least 3 mm beyond the folds. The portions contain a distal edge of the ear. The portions are located substantially symmetrically about the longitudinal centerline. Moreover, the transversely extending fold is spaced substantially equally between the opposing transverse end edges.

Also disclosed is a disposable absorbent article including a longitudinal centerline, a transverse center line, side margins, opposing transverse end edges, a front region, a rear region and a crotch region. The crotch region extends between and connects the front region and the rear region. The folded disposable absorbent article includes a pair of ears associated with the rear region. The disposable absorbent article includes at least a first longitudinally extending fold in each side margin and one transversely extending fold. The first longitudinally extending fold has a width in the front region and a width in the rear region. The width in the front region is narrower than the width in the rear region. The ears are inboard of the first longitudinally extending fold in the rear region. At least a portion of each of the ears extends beyond the first longitudinally extending fold in each side margin in the front region. The portions of the ears are colored to connote the portions location. The portions extend at least 3 mm beyond the folds in the front region. The portions contain a distal edge of the ear. The portions are located substantially symmetrically about the longitudinal centerline. Moreover, the transversely extending fold is spaced substantially equally between the opposing transverse end edges.

### Drawings

The foregoing and other features, aspects and advantages of the present invention will become better understood with regard to the following description, appended claims and accompanying drawings where:
FIG. 1 illustrates a plan view of a disposable absorbent article in an unfolded, flat-out, uncontracted state (*i*.*e*., with all elastic induced gathering and contraction removed), with the bodyfacing surface of the article facing the viewer and with portions of the article partially cut away to illustrate underlying features;
FIG. 2 illustrates a plan view of a disposable absorbent article in an unfolded, flat-out, uncontracted state, with the garment facing surface of the article facing the viewer and with portions of the article partially cut away to illustrate underlying features;
FIG. 3 illustrates a plan view of one version of an ear suitable for incorporation into a disposable absorbent article;
FIG. 4 illustrates a plan view of a disposable absorbent article in an unfolded, flat-out, uncontracted stated, with the bodyfacing surface of the article facing the viewer;
FIG. 5 illustrates an end view of the disposable absorbent article of FIG. 4, when viewed from a transverse end edge;
FIG. 6 illustrates a plan view of a disposable absorbent article with the longitudinal side edges being folded in toward the longitudinal centerline;
FIGs. 7A, 7B and 7C illustrate representative transitions suitable for use in a disposable absorbent article;
FIG. 8 illustrates a plan view of a folded disposable absorbent article;
FIG. 9 illustrates an end view of the disposable absorbent article of FIG. 8, when viewed from a transverse end edge;
FIG. 10 illustrates a plan view of a folded disposable absorbent article;
FIG. 11 illustrates an end view of the disposable absorbent article of FIG. 10, when viewed from a transverse end edge;
FIG. 12 illustrates a plan view of a folded disposable absorbent article;
FIG. 13 illustrates an end view of the disposable absorbent article of FIG. 12, when viewed from a transverse end edge;
FIG. 13A illustrates an end side view of a disposable absorbent article, when viewed from a transverse end edge;
FIG. 14 illustrates a plan view of the disposable absorbent article of FIG. 8, 10 or 12 after additional folding;
FIG. 15 illustrates a plan view of a folded disposable absorbent article;
FIG. 16 illustrates a plan view of the disposable absorbent article of FIG. 15 after additional folding;
FIG. 17 illustrates a plan view of a folded disposable absorbent article;
FIG. 18 illustrates a plan view of the disposable absorbent article of FIG. 17 after additional folding;
   and
FIG. 19 illustrates a package of absorbent articles with portions of the package partially cut away to illustrate the disposable absorbent articles therein.

### Description

The present invention relates folded disposable absorbent articles and methods of folding disposable absorbent articles. While the various versions of the present invention are described in terms of a disposable absorbent article such as an infant diaper, the invention is equally applicable to other disposable absorbent articles such as adult incontinence garments.

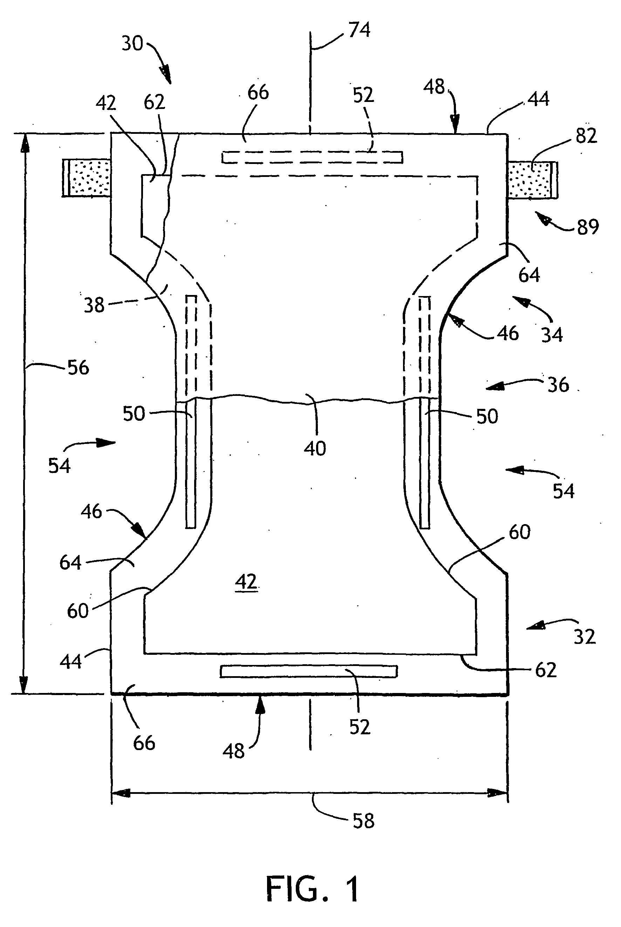

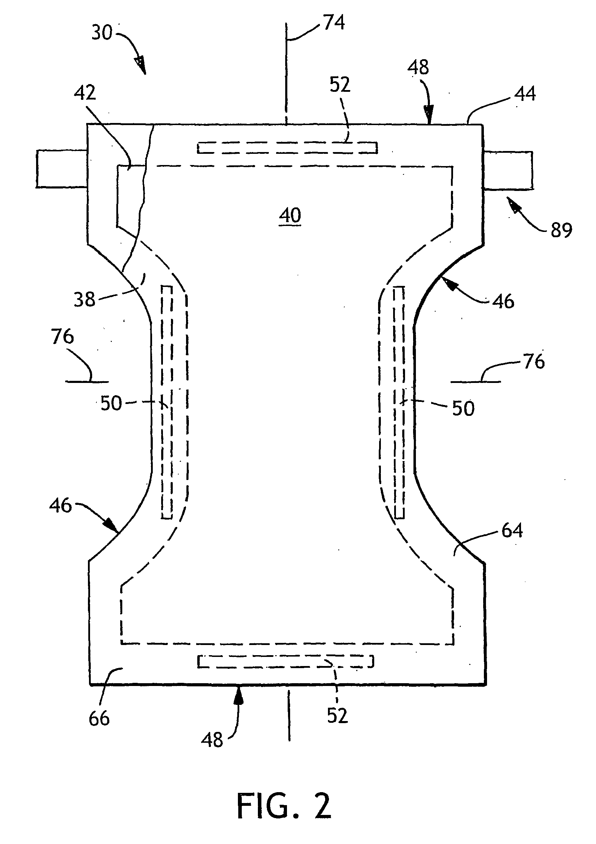

Referring now to the drawings, FIG. 1 illustrates a disposable absorbent article such as a disposable diaper (30) in an unfolded, flat-out, uncontracted state (*i*.*e*., with all elastic induced gathering and contraction removed). Portions of the structure are partially cut away to more clearly show the interior construction of the diaper (30), with the surface of the diaper (30) which contacts the wearer facing the viewer. FIGs. 1 and 2 illustrate a disposable diaper (30) as having a front region (32), a rear region (34) and a crotch portion (36) located between the front and rear regions. The diaper (30) includes a backsheet (38), a topsheet (40), and an absorbent core (42) situated between the backsheet and the topsheet. The outer edges of the diaper (30) define a periphery (44) with transversely opposed, longitudinally extending side edges (46); longitudinally opposed, transversely extending end edges (48); and a system of elastomeric gathering members, such as a system including leg elastics (50) and waist elastics (52). The longitudinal side edges (46) define the leg openings (54) for the diaper (30), and optionally, are curvilinear and contoured. The transverse end edges (48) are illustrated as straight, but optionally, may be curvilinear. The diaper (30) may also include additional components to assist in the acquisition, distribution and storage of bodily waste. For example, the diaper (30) may include a transport layer, such as described in U.S. Patent No. 4,798,603, issued to Meyer et al., or a surge management layer, such as described in European Patent Application Publication No. 0 539 703, published May 5, 1993.

With regard to the designated surfaces of a disposable absorbent article and its components, the various upper or bodyfacing surfaces are configured to face toward the body of the wearer when the absorbent article is worn by the wearer for ordinary use. The various opposing or lower surfaces are configured to face away from the wearer's body when the absorbent article is worn by the wearer.

The diaper (30) generally defines a longitudinally extending length dimension (56), and a laterally extending width dimension (58), as representatively illustrated in FIG. 1. The diaper (30) may have any desired shape, such as rectangular, I-shaped, a generally hourglass shape, or a T-shape.

The backsheet (38) defines a length and a width which, in the illustrated version, coincide with the length and width of the diaper (30). The absorbent core (42) generally defines a length and width which are less than the length and width of the backsheet (38), respectively. Thus, marginal portions of the diaper (30), such as marginal sections of the backsheet (38), may extend past the transversely opposed, longitudinally extending terminal side edges (60) and/or the longitudinally opposed, transversely extending terminal end edges (62) of the absorbent core (42) to form side margins (64) and end margins (66) of the diaper (30). Alternatively, the marginal portions of the diaper (30), specifically the side margins (64) and the end margins (66), may extend inwardly from the periphery (44) of the diaper (30). The topsheet (40) is generally coextensive with the backsheet (38), but may optionally cover an area which is larger or smaller than the area of the backsheet, as desired. The backsheet (38) and topsheet (40) are intended to face the garment and body of the wearer, respectively, while in use. As used herein when describing the topsheet (40) in relation to the backsheet (38) and vice versa, the term "associated" encompasses configurations in which the topsheet is directly joined to the backsheet, and configurations where the topsheet is indirectly joined to the backsheet by affixing portions of the topsheet to intermediate members which in turn are affixed to at least portions of the backsheet. The topsheet (40) and the backsheet (38) can, for example, be joined to each other in at least a portion of the diaper periphery (44) by attachment mechanisms (not shown) such as adhesive bonds, sonic bonds, thermal bonds, pinning, stitching, or a variety of other attachment techniques known in the art, as well as combinations thereof.

The topsheet (40) suitably presents a bodyfacing surface which is compliant, soft feeling, and non-irritating to the wearer's skin. Further, the topsheet (40) may be less hydrophilic than the absorbent core (42), to present a relatively dry surface to the wearer, and is sufficiently porous to be liquid permeable, permitting liquid to readily penetrate through its thickness. A suitable topsheet (40) may be manufactured from a wide selection of web materials, such as porous foams, reticulated foams, apertured plastic films, natural fibers, synthetic fibers (for example, polyester or polypropylene fibers), or a combination of natural and synthetic fibers. The topsheet (40) is suitably employed to help isolate the wearer's skin from liquids held in the absorbent core (42).

Various woven and nonwoven fabrics may be used for the topsheet (40). For example, the topsheet (40) may be composed of a meltblown or spunbonded web of polyolefin fibers. The topsheet (40) may also be a bonded-carded web composed of natural and/or synthetic fibers. The topsheet (40) may be composed of a substantially hydrophobic material, and the hydrophobic material may, optionally, be treated with a surfactant, or otherwise processed, to impart a desired level of wettability and hydrophilicity. Specifically; the topsheet (40) may be a nonwoven, spunbond, polypropylene fabric composed of about 2.8 to about 3.2 denier fibers formed into a web having a basis weight of about 22 gsm and a density of about 0.06 g/cc.

The topsheet (40) may also be surface treated with about 0.3 weight percent of a surfactant mixture that contains a mixture of AHCOVEL Base N-62 surfactant and GLUCOPON 220UP surfactant in about a 3:1 ratio based on a total weight of the surfactant mixture. The AHCOVEL Base N-62 surfactant is purchased from Hodgson Textile Chemicals Inc., a business having offices in Mount Holly, North Carolina, and includes a blend of hydrogenated ethoxylated castor oil and sorbitan monooleate in a 55:45 weight ratio. The GLUCOPON 220UP surfactant is purchased from Henkel Corporation, Gulph Mills, Pennsylvania, and includes alkyl polyglycoside. The surfactant may also include additional ingredients such as aloe. The surfactant may be applied by any conventional means, such as spraying, printing, brush coating, foam or the like. The surfactant may be applied to the entire topsheet (40) or may be selectively applied to particular sections of the topsheet, such as the medial section along the longitudinal centerline of a diaper, to provide greater wettability of such sections.

The backsheet (38) may suitably be composed of a material which is either liquid permeable or liquid impermeable. It is generally desirable that the backsheet (38) be formed from a material which is substantially liquid impermeable. For example, a typical backsheet (38) can be manufactured from a thin plastic film or other flexible liquid impermeable material. Moreover, the backsheet (38) may be formed from a polyethylene film having a thickness of from about 0.012 mm (0.5 mil) to about 0.051 mm (2.0 mils). If desirous of presenting the backsheet (38) with a more cloth-like feel, the backsheet may include a polyethylene film having laminated to the lower or opposing surface thereof a nonwoven web, such as a spunbond web of polyolefin fibers. For example, a polyethylene film having a thickness of about 0.015 mm (0.6 mil) may have thermally laminated thereto a spunbond web of polyolefin fibers, which fibers have a thickness of about 1.5 to about 2.5 denier per filament, which nonwoven web has a basis weight of about 24 gsm (0.7 osy). Methods of forming such cloth-like outer covers are known to those skilled in the art.

Further, the backsheet (38) may be formed of a woven or nonwoven fibrous web layer which has been totally or partially constructed or treated to impart a desired level of liquid impermeability to selected regions that are adjacent or proximate the absorbent core (42). Still further, the backsheet (38) may optionally be composed of micro-porous "breathable" material which permits vapors to escape from the absorbent core (42) while still preventing liquid exudates from passing through the backsheet.

The absorbent core (42) may include a matrix of hydrophilic fibers, such as a web of cellulosic fluff, mixed with particles of a high-absorbency material commonly known as superabsorbent material. In a particular version, the absorbent core (42) includes a mixture of superabsorbent hydrogel-forming particles and wood pulp fluff. The wood pulp fluff may be exchanged with synthetic polymeric, meltblown fibers or with a combination of meltblown fibers and natural fibers. The superabsorbent particles may be substantially homogeneously mixed with the hydrophilic fibers or may be non-uniformly mixed. The absorbent core (42) may be any suitable structure for absorbing and/or retaining exudates.

The absorbent core (42) may have any of a number of shapes. For example, the absorbent core (42) may be rectangular, I-shaped or T-shaped. It is often considered as desirable for the absorbent core (42) to be narrower in the crotch portion than the rear or front region(s).

The high-absorbency material can be selected from natural, synthetic and modified natural polymers and materials. The high-absorbency materials can be inorganic materials, such as silica gels, or organic compounds, such as crosslinked polymers. The term "crosslinked" refers to any means for effectively rendering normally water-soluble materials substantially water insoluble, but swellable. Such means can include, for example, physical entanglement, crystalline domains, covalent bonds, ionic complexes and associations, hydrophilic associations, such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrolidone), poly(vinyl morpholinone), poly(vinyl alcohol), and mixtures and copolymers thereof. Further polymers suitable for use in the absorbent core include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthum gum, locust bean gum, and the like. Mixtures of natural and wholly or partially synthetic absorbent polymers can also be useful. Processes for preparing synthetic, absorbent gelling polymers are disclosed in U.S. Patent No. 4,076,663, issued to Masuda et al., and U.S. Patent No. 4,286,082, issued to Tsubakimoto et al.

The high-absorbency material may be in a variety of geometric forms. It is desired that the high-absorbency material be in the form of discrete particles. Howeyer, the high-absorbency material may also be in the form of fibers, flakes, rods, spheres, needles, or the like. Often, the high-absorbency material is present in the absorbent core (42) in an amount of from about 5 to about 100 weight percent based on total weight of the absorbent core.

The disposable absorbent articles described herein also include fasteners (82) for securing the absorbent article about the waist of the wearer. The illustrated versions of the diaper (30) include such fasteners (82). In at least one version, the fasteners (82) are situated in the rear region (34) of the diaper (30), and located inboard each longitudinal extending side edge (46). The fasteners (82) may be configured to encircle the hips of the wearer and engage the backsheet (38) of the front region (32) of the diaper (30) for holding the diaper (30) on the wearer. Suitable fasteners are well known to those of skill in the art and can include adhesive tape tab fasteners, hook and loop fasteners, mushroom fasteners, snaps, pin, belts and the like, and combinations thereof. Desirably, the fasteners (82) are releasably engageable directly with the garment facing surface of the backsheet (38). Alternatively, the diaper (30) may include a fastening panel (not illustrated) situated in the front region (32) of the garment facing surface of the backsheet (38). In such a configuration, the fasteners (82) are releasably engageable with the fastening panel to maintain the diaper (30) about the waist of the wearer. As representatively illustrated in FIGs. 1 - 4, the fasteners (82) may be hook type fasteners and the backsheet (38) may be configured to function as a complimentary loop type fastener. Desirably, the fasteners (82) are hook type fasteners which are releasably engageable with the backsheet (38). Such an arrangement provides the ability to vary the size of the waist opening in very small increments over a wide range to fit the waist of the wearer. The fasteners (82) may have a variety of shapes and sizes which provide the desired fastening of the diaper (30) about the waist of the wearer.

The term "inboard" is intended to refer to the direction from a periphery or an edge toward a respective centerline. The term "outboard" is intended to refer to a direction away from a respective centerline.

The diaper (30) includes ears (89). In particular arrangements, each ear (89) extends laterally at the opposed, lateral ends of at least one waist region of the backsheet (38), to provide terminal side sections of the article. In addition, each ear (89) may substantially span from a laterally extending, terminal waistband edge to approximately the location of its associated and corresponding leg opening section of the diaper.

In the various configurations of the invention, the ears (89) may be integrally formed with a selected diaper component. For example, ears (89) can be integrally formed from the layer of material which provides the backsheet (38), or may be integrally formed from the material employed to provide the topsheet (40). In alternative configurations, the ears (89) may be provided by one or more separately provided members that are connected and assembled to the backsheet (38), to the topsheet (40), in between the backsheet and topsheet, or in various fixedly attached combinations of such assemblies.

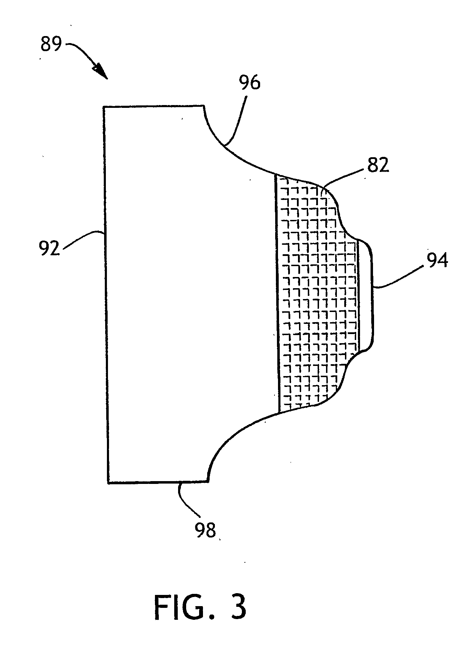

As illustrated in FIG. 3, the ear (89) may be provided by a separately provided member that includes a proximal edge (92), an opposed distal edge (94), a first connecting edge (96), and a second connecting edge (98). As used herein, the proximal edge (92) is that edge of the ear (89) which is connected or otherwise associated in an operable manner to a longitudinal extending side edge (46) of the diaper (30). The distal edge (94) is that edge of the ear (89) which is opposite the proximal edge (92) moving in a direction outboard from a longitudinal centerline (74) of the diaper (30). The first (96) and second (98) connecting edges connect the proximal edge (92) and the distal edge (94) thereby defining a body of material which at least partially defines an ear (89). The ear (89) may be made of a variety of materials including those that are extensible, elastomeric and/or non-elastomeric. As used herein when describing the diaper (30) in relation to the ear (89) and vice versa, the term "associated" encompasses configurations in which the diaper (30) is directly joined to the ear (89), and configurations wherein the diaper (30) is indirectly joined to the ear (89) by affixing the diaper (30) to intermediate members which in turn are affixed to the ear (89). ). As used herein, the ear (89) forms at least a portion of the side margin (64).

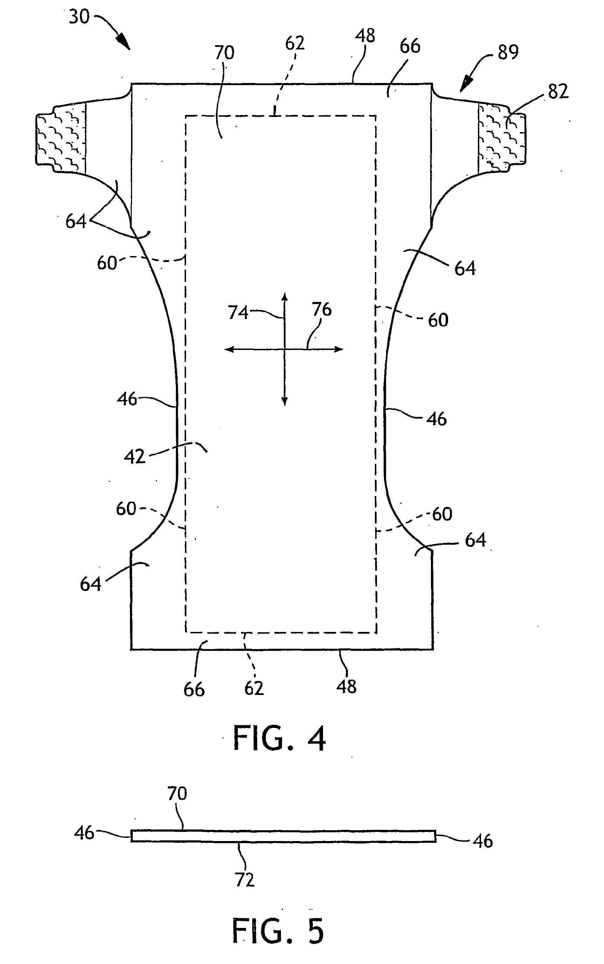

Referring now to FIGs.4 and 5, in its unfolded condition a disposable absorbent article, in this instance a diaper (30), has an upper surface (70), a lower surface (72), a longitudinal centerline (74), a transverse centerline (76), opposing longitudinal side edges (46), opposing transverse end edges (48), side margins (64), opposing longitudinal terminal side edges (60) of the absorbent core (42), and opposing transverse terminal end edges (62) of the absorbent core (42.

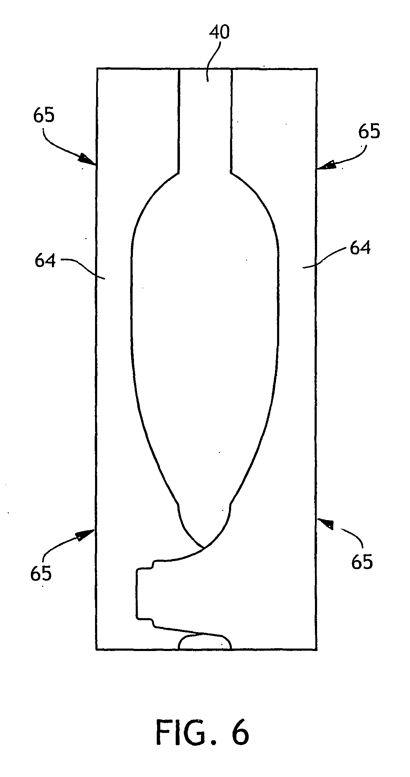

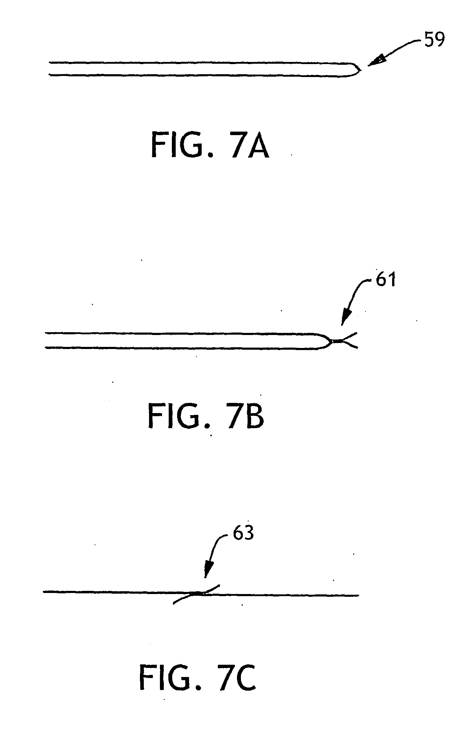

Referring now to FIG. 6, in accordance with the invention, the diaper (30) is folded by forming at least a first longitudinally extending fold (65) in each of the side margins (64). The first longitudinally extending folds (65) are usually formed by folding the first longitudinal side edges (46) inward toward the initial upper surface (70) and thus bringing at least a portion of the initial upper surface into facing relationship with another portion of the initial upper surface. Each first longitudinally extending fold (65) so formed is typically inboard of the original positioning of the respective first longitudinal side edge (46) and either on or outboard of the respective longitudinal terminal side edge (60) of the absorbent core (42). Typically, the initial surface (70) is the topsheet (40); however, one of skill in the art will readily appreciate that the initial upper surface may alternatively be the backsheet (38). The diaper (30) may be further folded by forming a number of folds in each of the side margins (64). The diaper (30) may also be further folded by forming transversely extending folds. As used herein a fold includes any transition where two portions of the diaper (30) are doubled over upon themselves. Three types of transitions are illustrated in FIGs. 7A, 7B and 7C. The first transition, illustrated in FIG. 7A, is a 180 degree bend (59) of a single piece of material, this is considered a fold. The second transition, illustrated in FIG 7B, is a butt seam (61) where two pieces of material are joined together where the major free surfaces of the two pieces of material extend from the same side of the seam, this is considered a fold. The third transition, illustrated in FIG 7C, is an overlap seam (63) where two pieces of material are joined together where the major free surfaces of the two pieces of material extend from opposite sides of the overlap seam (63), this is not considered a fold since portions of the diaper are not doubled over upon themselves.

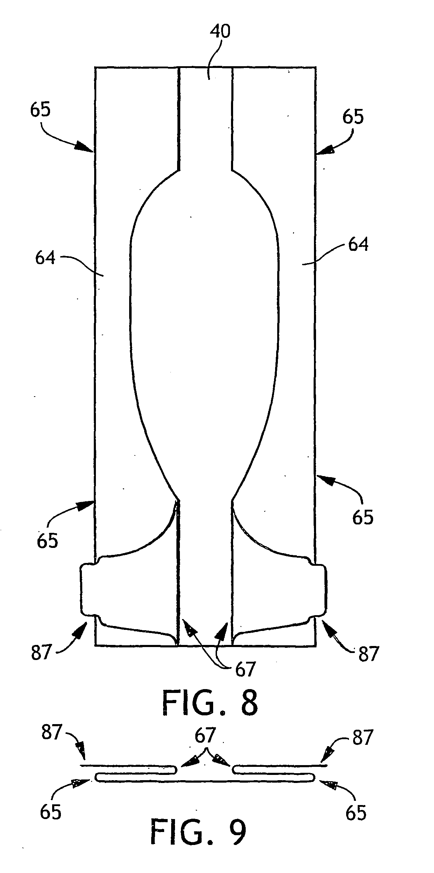

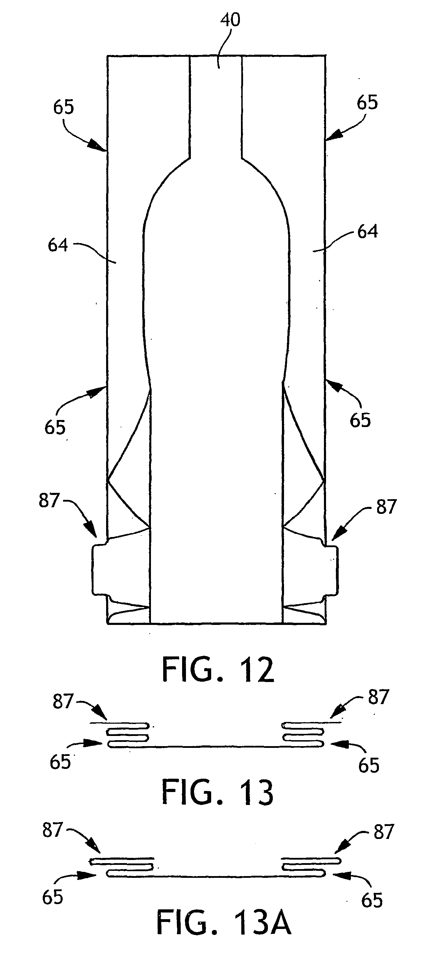

Referring now to FIG. 8, in accordance with the invention, the diaper (30) is further folded by forming at least a second longitudinally extending fold (67) in each of the side margins (64). The second longitudinally extending folds (67) may be formed by folding the longitudinal side edges (46) outward, away from the longitudinal centerline (74). The second longitudinally extending fold (67) is located such that an exposed portion (87) of the ear (89) extends beyond a portion of the first longitudinally extending fold (65). The exposed portion (87) of the ear (89) that extend beyond a portion of the first longitudinally extend fold (65) may be the proximal edge (92) as shown in FIGs 8, 9, 10, 11, 12, 13 and 16. Alternatively the ear may be folded such that the proximal edge (92) is in inboard the first longitudinally extending fold, and the exposed portion (87) of the ear that extends beyond a portion of the first longitudinally extending fold (65) is not the proximal edge (92) as shown in FIG. 13A. FIG. 9 illustrates an end view of the disposable absorbent article of FIG. 8, when viewed from a transverse end edge (48). It will be appreciated that the first longitudinally extending fold (65) and the second longitudinally extend fold (67) can be made in either order or simultaneously.

The diaper (30) may be folded such that the exposed portions (87) are symmetric about the longitudinal center line (74) (as shown), or alternatively, the diaper (30) may be folded such that the exposed portions (87) are asymmetric about the longitudinal centerline (74) (not shown).

Several benefits are provided to the user when an exposed portion (87) of the ear (89) extends beyond a portion of the first longitudinally extending fold (65). Primary is ease of opening. When a user opens a diaper (30) for donning, the user may open the diaper (30) to a substantially flat-out configuration. To accomplish this, the user may first locate the longitudinally extending side edges (46) and the transversely extending end edges (48). When an exposed portion (87) of the ear (89) extends beyond a portion of the first longitudinally extending fold (65), the number steps required to open the diaper (30) may be reduced, or the individual steps may be easier. Specifically, the ear (89) is relatively easier to find when an exposed portion (87) of the ear (89) extends beyond a portion of the longitudinally extending fold (65), as in FIG. 8, than it is to find when it does not extend beyond the longitudinally extending fold (65), as in FIG. 7. Further, in some configurations, when the user grasps the exposed portion (87) and pulls, the diaper (30) snaps open, such that in one "pull" the diaper (30) unfolds into a usable configuration.

Another benefit provided to the user when an exposed portion (87) of the ear (89) extends beyond a portion of the first longitudinally extending fold (65), is ease of diapering. The user may choose to place the diaper (30) under a child or incontinent adult, before extending the ear (89). The user is then able to access the exposed portion (87) of the ear (89) with out lifting or reaching under the back of the child or adult on whom the article is being donned.

In addition to extending beyond a portion of the first longitudinally extending fold (65), the exposed portion (87) of the ear (89) may have additional features that provide for ease of opening. The exposed portion (87) may include an indicator means for connoting the location of the exposed portion (87) This indicator means my be a color that visually sets the exposed portion (87) apart from the rest of the diaper (30) or area of the diaper (30) in close proximity to the exposed portion (87). The indicator means may be a tactile feature, such as embossing. The exposed portion (87) may be phosphorescent, such that the exposed portion (87) is easy to find when diapering in the dark.

Desirably, the exposed portion (87) of the ear (89) extends beyond a portion of the first longitudinally extending fold (65) no less then 2 mm, alternatively, no less than 7 mm; alternatively, no less than 10 mm; alternatively, no less than 15 mm; alternatively, no less than 20 mm; alternatively, no less than 27 mm; alternatively, no less than 32 mm; alternatively, no less than 36 mm; alternatively, no less than 40 mm; alternatively, no less than 43 mm; alternatively, no less than 48 mm; alternatively, no less than 53 mm; alternatively, no less than 58 mm; alternatively, no less than 60 mm; alternatively, no less than 64 mm; alternatively, no less than 67 mm; alternatively, no less than 71 mm; and finally, alternatively, no less than 75. Desirably, the exposed portion (87) of the ear (89) extends beyond a portion of the first longitudinally extending fold (65) no more than 80 mm; alternatively, no more than 75 mm; alternatively, no more than 71 mm; alternatively, no more than 67 mm; alternatively, no more than 64 mm; alternatively, no more than 63 mm; alternatively, no more than 60 mm; alternatively, no more than 53 mm; alternatively, no more than 48 mm; alternatively, no more than 43 mm; alternatively, no more than 40 mm; alternatively, no more than 36 mm; alternatively, no more than 32 mm; alternatively, no more than 27 mm; alternatively, no more than 24 mm; alternatively, no more than 20 mm; alternatively, no more than 16 mm; alternatively, no more than 13 mm; alternatively, no more than 10 mm; alternatively, no more than 7 mm; alternatively, no more than 6 mm; alternatively, no more than 5 mm; alternatively, no more than 4 mm; and finally, alternatively, no more than 3 mm. Thus, the exposed portion (87) of the ear (89) extends beyond a portion of the first longitudinally extending fold (65) typically is no less than 2 mm and no more than 80 mm; although the approximate length may vary according to, *inter alia,* the general design and intended use of the disposable absorbent article.

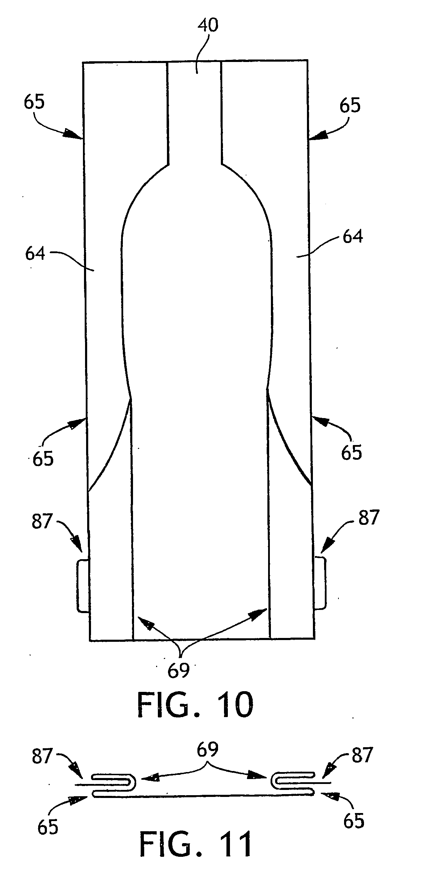

As shown in FIG. 10, a diaper (30) as illustrated in FIG. 8 may be further folded by forming at least one tertiary longitudinally extending fold (69) in each of the side margins (64). The tertiary longitudinally extending folds (69) may be formed by folding the second longitudinally extending folds (67) outward, away from the longitudinal centerline (74) resulting in the diaper (30) of FIG. 10. A diaper (30) folded in this manner provides an added advantage to the user in that during the donning process, the diaper (30) may be placed under the child or incontinent adult with a smaller amount of the ear (89) located under the child.

Alternatively, a diaper (30) may be folded by forming at least a first longitudinally extending fold (65) in each of the side margins (64), as illustrated in FIG. 7. The side margins (64) may then be folded further, in an accordion like manner, first with an outward fold followed by an inward fold, and so on. The accordion folds may be substantially uniformly spaced initially, ending with a longer length which results in the opposed distal edge (94) of the ear (89) extending beyond a portion of the first longitudinally extending fold (65), as illustrated in FIGs. 12 and 13. Alternatively, the accordion folds may be spaced such that an intermediate fold extends beyond a portion of the first longitudinally extending fold (65) and is thereby the exposed portion (87) of the ear (89) that extends beyond a portion of the first longitudinally extending fold (65), as illustrated in FIG. 13A.

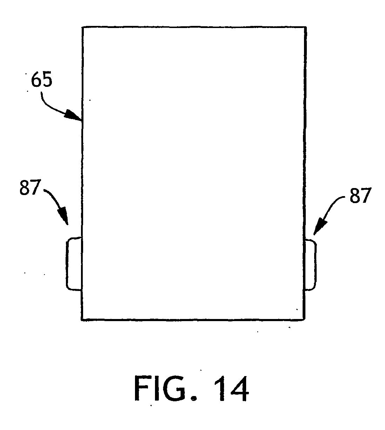

The diaper (30) folded as illustrated in FIGs. 8, 10 or 12 may be folded by forming one transversely extending fold. The transversely extending fold is formed by bringing a portion of the upper surface (70) into facing relationship with another portion of the upper surface (70), an example of which is illustrated in FIG. 14. The transversely extending fold runs generally parallel to the transverse centerline (76) of the diaper (30). Moreover, the transversely extending fold may be spaced substantially equally between the transverse end edges (48) or unequally between the transverse end edges. Typically, the initial upper surface (70) is the topsheet (40); however, one of skill in the art will readily appreciate that the initial upper surface may alternatively be the backsheet (38).

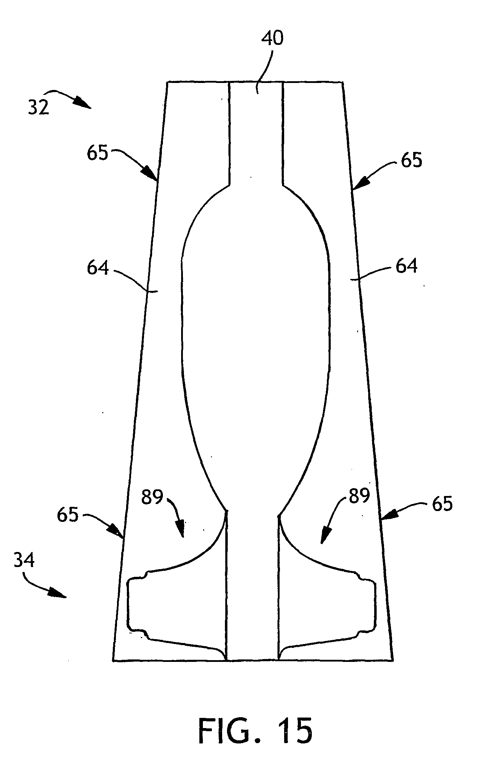

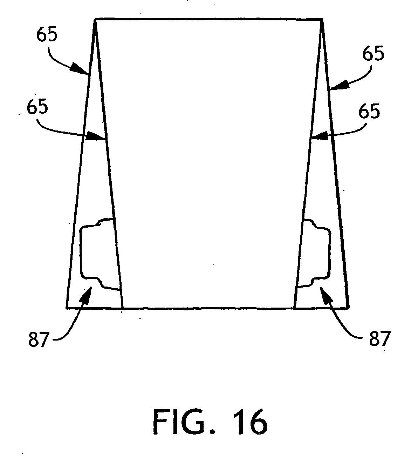

Alternatively, a diaper (30) may be folded by forming at least a first longitudinally extending fold (65) in each of the side margins (64). As illustrated in FIG. 15, which is not in accordance with the invention, the first longitudinally extending folds (65) may be non-parallel, such that the distance between the first longitudinally extending folds (65) in the front region (32) is different than the distance between the first longitudinally extending folds (65) in the rear region (34). In FIG. 15, the ears (89) are located in the rear region (34). Further the first longitudinally extending folds (65) are non-parallel such that the distance between the first longitudinally extending folds (65) in the rear region is greater than the distance between the first longitudinally extending folds (65) in the front region. The side margins (65) may be folder further such that the entire ear (89) is inbound of the first longitudinally extending folds (65) in the rear region (34). When the diaper (30) is folded in a transverse direction generally parallel to the transverse centerline (76), the first longitudinally extending folds (65) in the front region (32) overlays the rear region (34) and more specifically the ears (89), as illustrated in FIG. 16. This arrangement is not in accordance with the invention. In this way, an exposed portion (87) of the ear (89) extends beyond a portion of the first longitudinally extending fold (65). More specifically an exposed portion (87) of the ear (89) located in the rear region (34) extends beyond a portion of the first longitudinally extending fold (65) located in the front region (32). This alternatively folded diaper (30) provides benefits to the user such as ease of opening and ease of diapering as described herein. Additionally, this alternatively folded diaper (30) provides a benefit in the packaging process in that a portion of the first longitudinally extending fold (65) protects the exposed portions (87).

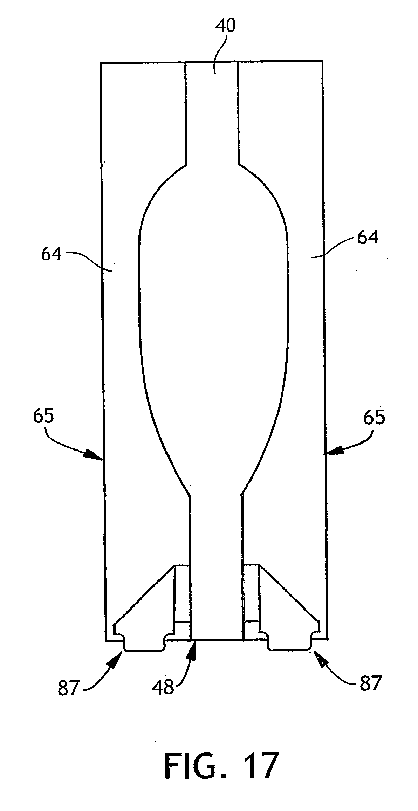

In other arrangements not in accordance with the invention, a diaper (30) may be folded such that an exposed portion (87) of the ear (89) extends beyond a transverse end edge (48). The diaper (30) may be folded by forming at least a first longitudinally extending fold (65) in each of the side margins (64). The side margins (64) may then be folded one or more times, at an angle to the first longitudinally extending fold (65), such that an exposed portion (87) of the ear (89) extends beyond a transverse end edge (48). As illustrated in FIG. 17, the side margins (64) have been folded twice. First with a first longitudinally extending fold (65) and second with a fold approximately 45 degrees to the first longitudinally extending fold (65). The number and angle of the folds in the side margins (64) may need to be modified depending on the size and shape of the side margins (64). This alternatively folded diaper (30) provides the benefit to the user of ease of opening as described herein.

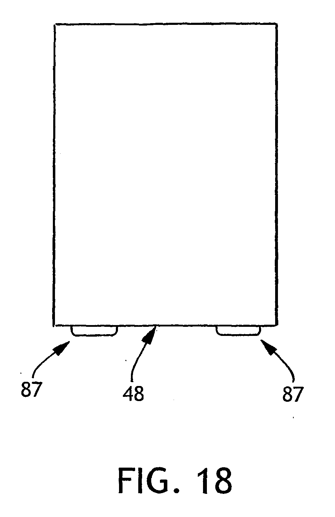

The diaper (30) folded as illustrated in FIG. 17 may be further folded by forming one fold extending in a transverse direction, as described above, resulting in the diaper illustrated in FIG. 18.

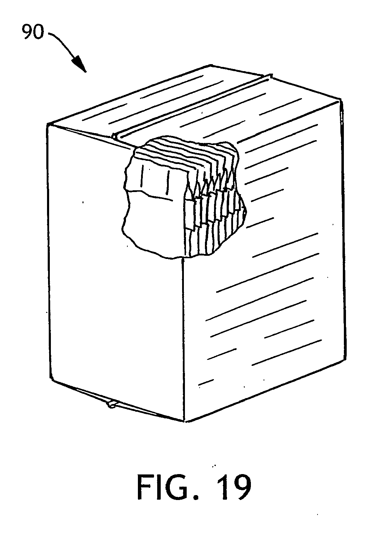

In another aspect, the present invention provides a package (90) of the disposable absorbent articles described above, as shown in FIG. 19. The package includes a container such as, for example, a plastic bag, and at least one disposable absorbent article.

Having described the invention in rather full detail, it will be readily apparent that various changes and modifications can be made without departing from the invention. All of such changes and modifications are contemplated as being within the scope of the invention as defined by the appended claims.

## Claims

1. A method of folding a disposable absorbent article (30), the article having a longitudinal centerline, transversely spaced side margins (64) including an unfolded configuration, a front region (32), a rear region (34) and a crotch region (36) which extends between and connects the front region and the rear region, and a pair of ears (89) associated with the side margins in at least one of the front and rear regions;
the method of folding comprising:
forming first and second longitudinally extending folds (65, 67) in each side margin, **characterized in that** the first and second longitudinally extending folds are configured such that at least a portion (87) of each of the ears extend transversely beyond at least a portion of the first longitudinally extending fold in each side margin, and wherein at least a portion of each of the ears extends transversely beyond all longitudinally extending folds in the side margins.

2. The method described in claim 1 further comprising forming one transversely extending fold in the absorbent article, the one fold being spaced between opposing transverse end edges and desirably equally between the transverse end edges.

3. The method described in claim 1, further comprising forming at least a third fold (69) in each of the side margins.

4. The method described in claim 1, wherein the side margins are folded with accordion folds.

5. A folded disposable absorbent article having a longitudinal centerline, transversely spaced side margins, a front region (32), a rear region (34) and a crotch region (36) which extends between and connects the front region and the rear region, and a pair of ears (89) associated with the side margins in at least one of the front and rear regions, and first and second longitudinally extending folds in each side margin, **characterized in that** at least a portion of each of the ears extends transversely beyond at least a portion of the first longitudinally extending fold in each side margin, wherein at least a portion of each of the ears extends transversely beyond all longitudinally extending folds in the side margins.

6. The method or folded disposable absorbent article of any preceding claim, wherein the first longitudinally extending fold is a 180 degree bend or a seam.

7. The method or folded disposable absorbent article of any preceding claim, wherein the portion of the pair of ears include indicator means for connoting the ears location.

8. The method or folded disposable absorbent article of claim 7, wherein the indicator means comprises color or embossing.

9. The method or folded disposable absorbent article of any preceding claim, wherein at least a portion of each of the ears extends transversely beyond the longitudinally extending fold at least 3 mm and desirably at least 10 mm.

10. The method or folded disposable absorbent article of any preceding claim, wherein the portion of the ear contains a distal edge (94) of the ear.

11. The method or folded disposable absorbent article of claim 10, wherein the pair of ears (89) are bonded to the absorbent article (30) in the rear region.

12. The method or folded disposable absorbent article of any preceding claim, wherein the portions of each of the ears are located substantially symmetrically about the longitudinal centerline.

13. A package of folded disposable absorbent articles comprising a container and at least one folded disposable absorbent article of claim 5, or at least one disposable absorbent article folded according to the method of claim 1.

14. The folded disposable absorbent article of claim 5, the disposable absorbent article including at least a first, second and third longitudinally extending folds in each side margin and one transversely extending fold, wherein at least a portion of each of the ears extends beyond the first, second and third longitudinally extending folds in each side margin, wherein the portions of the ears are colored to connote the portions location, the portions extend at least 3 mm beyond the folds, the portions contain a distal edge of the ear, the portions are located substantially symmetrically about the longitudinal centerline, and the transversely extending fold is spaced substantially equally between the opposing transverse end edges.

15. The folded disposable absorbent article of claim 5, wherein the pair of ears is associated with the rear region, the disposable absorbent article including at least one transversely extending fold, wherein the first longitudinally extending fold has a width in the front region and a width in the rear region, the width in the front region is narrower than the width in the rear region, the ears are inboard of the first longitudinally extending fold in the rear region, at least a portion of each of the ears extends beyond the first longitudinally extending fold in each side margin in the front region, the portions of the ears are colored to connote the portions location, the portions extend at least 3 mm beyond the folds in the front region, the portions contain a distal edge of the ear, the portions are located substantially symmetrically about the longitudinal centerline, and the transversely extending fold is spaced substantially equally between the opposing transverse end edges.

16. The method or folded absorbent article of any preceding claim, the article comprising transversely opposed, longitudinally extending side edges (46), a backsheet (38), a topsheet (40), and an absorbent core (42) situated between the backsheet and the topsheet, the absorbent core (42) having transversely opposed, longitudinally extending terminal side edges (60), wherein said first longitudinally extending folds (65) are formed by folding the first longitudinal side edges (46) of the article inward toward an initial upper surface (70) of the article, each first longitudinally extending fold being formed inboard of the original positioning of the respective longitudinal side edge (46) and either on or outboard of the respective longitudinal terminal side edge (60) of the absorbent core (42), and the second longitudinally extending folds (67) being formed by folding the longitudinal side edges (46) of the article outward, away from the longitudinal centerline (74).

17. The method or folded absorbent article of any preceding claim, wherein the article further comprises fasteners (82) for securing the absorbent article about the waist of the wearer.

18. The method or article described in any preceding claim, wherein the disposable absorbent article (30) is a diaper.

19. The method or article described in any preceding claim, wherein the first longitudinally extending fold (65) is outboard of the second longitudinally extending fold (67) after folding.

## Patentansprüche

1. Ein Verfahren zum Falten eines absorbierenden Einwegartikels (30), wobei der Artikel eine longitudinale Mittellinie, Seitenränder mit Querabstand (65), die eine ungefaltete Konfiguration beinhalten, einen vorderen Bereich (32), einen hinteren Bereich (34) und einen Schrittbereich (36), der sich zwischen dem vorderen Bereich und dem hinteren Bereich erstreckt und diese verbindet, und ein Paar von Laschen (89) aufweist, die an den Seitenrändern in zumindest einem der vorderen und hinteren Bereiche angeschlossen sind;
wobei das Verfahren zum Falten umfasst:
Bilden einer ersten und zweiten sich longitudinal erstreckenden Falte (65, 67) in jedem Seitenrand, **dadurch gekennzeichnet, dass** die erste und zweite sich longitudinal erstreckende Falte konfiguriert sind, so dass sich zumindest ein Teil (87) jeder der Laschen quer über zumindest einen Teil der ersten sich longitudinal erstreckenden Falte in jedem Seitenrand erstreckt, und wobei sich zumindest ein Teil jeder der Laschen quer über alle sich longitudinal erstreckenden Falten in den Seitenrändern erstreckt.

2. Das Verfahren nach Anspruch 1, weiterhin umfassend Bilden einer sich quer erstreckenden Falte in dem absorbierenden Artikel, wobei die eine Falte zwischen entgegengesetzten Querendkanten beabstandet ist und wünschenswerterweise abstandsgleich zwischen den Querendkanten ist.

3. Das Verfahren nach Anspruch 1, weiterhin umfassend Bilden zumindest einer dritten Falte (69) in jedem der Seitenränder.

4. Das Verfahren nach Anspruch 1, wobei die Seitenränder mit Ziehharmonikafalten gefaltet sind.

5. Ein gefalteter, absorbierender Einwegartikel, der eine longitudinale Mittellinie, Seitenränder mit Querabstand, einen vorderen Bereich (32), einen hinteren Bereich (34) und einen Schrittbereich (36), der sich zwischen dem vorderen Bereich und dem hinteren Bereich erstreckt und diese verbindet, und ein Paar von Laschen (89), die an den Seitenrändern in zumindest einem der vorderen und hinteren Bereiche angeschlossen sind, und eine erste und eine zweite sich longitudinal erstreckende Falte in jedem Seitenrand aufweist, **dadurch gekennzeichnet, dass** sich zumindest ein Teil jeder der Laschen quer über zumindest einen Teil der ersten sich longitudinal erstreckenden Falte in jedem Seitenrand erstreckt, wobei sich zumindest ein Teil jeder der Laschen quer über alle sich longitudinal erstreckenden Falten in den Seitenrändern erstreckt.

6. Das Verfahren oder der gefaltete absorbierende Einwegartikel nach einem der vorangehenden Ansprüche, wobei die erste sich longitudinal erstreckende Falte ein 180 Grad Knick oder ein Saum ist.

7. Das Verfahren oder der gefaltete absorbierende Einwegartikel nach einem der vorangehenden Ansprüche, wobei der Teil des Paares von Laschen Anzeigemittel zum Konnotieren der Laschenposition beinhaltet.

8. Das Verfahren oder der gefaltete absorbierende Einwegartikel nach Anspruch 7, wobei das Anzeigemittel Farbe oder eine Prägung umfasst.

9. Das Verfahren oder der gefaltete absorbierende Einwegartikel nach einem der vorangehenden Ansprüche, wobei sich zumindest ein Teil jeder der Laschen zumindest 3 mm und wünschenswerterweise zumindest 10 mm quer über die sich longitudinal erstreckende Falte erstreckt.

10. Das Verfahren oder der gefaltete absorbierende Einwegartikel nach einem der vorangehenden Ansprüche, wobei der Teil der Laschen eine distale Kante (94) der Lasche enthält.

11. Das Verfahren oder der gefaltete absorbierende Einwegartikel nach Anspruch 10, wobei das Paar von Laschen (89) in dem hinteren Bereich an den absorbierenden Artikel (30) gebunden ist.

12. Das Verfahren oder der gefaltete absorbierende Einwegartikel nach einem der vorangehenden Ansprüche, wobei die Teile jeder der Laschen im Wesentlichen symmetrisch um die longitudinale Mittellinie angeordnet sind.

13. Eine Packung von gefalteten absorbierenden Einwegartikeln umfassend einen Behälter und zumindest einen gefalteten absorbierenden Einwegartikel nach Anspruch 5, oder zumindest einen absorbierenden Einwegartikel, der nach dem Verfahren des Anspruchs 1 gefaltet ist.

14. Der gefaltete absorbierende Einwegartikeln nach Anspruch 5, wobei der absorbierende Einwegartikel zumindest eine erste, zweite und dritte sich longitudinal erstreckende Falte in jedem Seitenrand und eine sich quer erstreckende Falte beinhaltet, wobei sich zumindest ein Teil jeder der Laschen über die erste, zweite und dritte sich longitudinal erstreckende Falte in jedem Seitenrand erstreckt, wobei die Teile der Laschen farbig sind, um die Position der Laschen zu konnotieren, wobei sich die Teile zumindest 3 mm über die Falten erstrecken, wobei die Teile eine distale Kante der Lasche enthalten, wobei die Teile im Wesentlichen symmetrisch um die longitudinale Mittellinie angeordnet sind, und die sich quer erstreckende Falte im Wesentlichen abstandsgleich zwischen den entgegengesetzten Querendkanten ist.

15. Der gefaltete absorbierende Einwegartikeln nach Anspruch 5, wobei das Paar von Laschen an den hinteren Bereich angeschlossen ist, wobei der absorbierende Einwegartikel zumindest eine sich quer erstreckenden Falte beinhaltet, wobei die erste sich longitudinal erstreckende Falte eine Breite im vorderen Bereich und eine Breite im hinteren Bereich aufweist, wobei die Breite im vorderen Bereich schmaler ist als die Breite im hinteren Bereich, wobei sich die Laschen innwärtig von der ersten sich longitudinal erstreckenden Falte befinden, wobei sich zumindest ein Teil jeder der Laschen über die erste sich longitudinal erstreckende Falte auf jedem Seitenrand im vorderen Bereich erstreckt, wobei die Teile der Laschen gefärbt sind, um die Position der Laschen zu konnotieren, wobei sich die Teile zumindest 3 mm über die Falten im vorderen Bereich erstrecken, wobei die Teile eine distale Kante der Lasche enthalten, wobei die Teile im Wesentlichen symmetrisch um die longitudinale Mittellinie positioniert sind, und die sich quer erstreckende Falte im Wesentlichen abstandsgleich zwischen den entgegengesetzten Querendkanten ist.

16. Das Verfahren oder der gefaltete absorbierende Einwegartikel nach einem der vorangehenden Ansprüche, wobei der Artikel quer entgegengesetzte, sich longitudinal erstreckende Seitenkanten (46), eine untere Schicht (38), eine obere Schicht (40) und einen absorbierenden Kern (42) umfasst, der zwischen der unteren Schicht und der oberen Schicht positioniert ist, wobei der absorbierende Kern (42) quer entgegengesetzte, sich longitudinal erstreckende, abschließende Seitenkanten (60) aufweist, wobei die ersten, sich longitudinal erstreckenden Falten (65) durch Falten der ersten longitudinalen Seitenkanten (46) des Artikels nach innen in Richtung einer anfänglich oberen Oberfläche (70) des Artikels gebildet werden, wobei jede erste sich longitudinal erstreckende Falte innwärtig von der ursprünglichen Positionierung der entsprechenden longitudinalen Seitenkante (46) gebildet wird und entweder auf der entsprechenden longitudinalen, abschließenden Seitenkante (60) oder außerhalb von der entsprechenden longitudinalen, abschließenden Seitenkante (60) des absorbierenden Kerns (42) gebildet wird, und die zweiten, sich longitudinal erstreckenden Falten (67) durch Falten der longitudinalen Seitenkanten (46) des Artikels nach außen, weg von der longitudinalen Mittellinie (74) gebildet werden.

17. Das Verfahren oder der gefaltete absorbierende Einwegartikel nach einem der vorangehenden Ansprüche, wobei der Artikel Befestigungsmittel (82) zum Befestigen des absorbierenden Artikels um den Bauch des Trägers umfasst.

18. Das Verfahren oder der gefaltete absorbierende Einwegartikel nach einem der vorangehenden Ansprüche, wobei der absorbierende Einwegartikel (30) eine Windel ist.

19. Das Verfahren oder der gefaltete absorbierende Einwegartikel nach einem der vorangehenden Ansprüche, wobei sich die erste sich longitudinal erstreckende Falte (65) nach dem Falten außerhalb von der zweiten, sich longitudinal erstreckenden Falte (67) befindet.

## Revendications

1. Procédé de pliage d'un article absorbant jetable (30), l'article ayant une ligne centrale longitudinale, des marges latérales transversalement espacées (64) incluant une configuration dépliée, une région avant (32), une région arrière (34) et une région d'entrejambe (36) qui s'étend entre la région avant et la région arrière et les connecte, et une paire d'oreilles (89) associées aux marges latérales dans l'une au moins des régions avant et arrière ;
le procédé de pliage comprenant :
la formation de premier et second plis qui s'étendent longitudinalement (65,67) dans chacune des marges latérales, **caractérisé en ce que** les premier et second plis qui s'étendent longitudinalement sont configurés de telle sorte qu'au moins une portion (87) de chacune des oreilles s'étend transversalement au-delà d'au moins une portion du premier pli qui s'étend longitudinalement dans chacune des marges latérales, au moins une portion de chacune des oreilles s'étendant transversalement au-delà de tous les plis qui s'étendent longitudinalement dans les marges latérales.

2. Procédé selon la revendication 1, comprenant, en outre, la formation d'un pli s'étendant transversalement dans l'article absorbant, ledit un pli étant à l'écart entre des bords d'extrémité transversaux opposés et, de préférence, équidistants des bords d'extrémité transversaux.

3. Procédé selon la revendication 1, comprenant, en outre, la formation d'au moins un troisième pli(69) dans chacune des marges latérales.

4. Procédé selon la revendication 1, dans lequel les marges latérales sont pliées en accordéon.

5. Article absorbant jetable plié ayant une ligne centrale longitudinale, des marges latérales transversalement espacées, une région avant (32), une région arrière (34) et une région d'entrejambe (36) qui s'étend entre la région avant et la région arrière et les connecte, et une paire d'oreilles (89) associées aux marges latérales dans l'une au moins des régions avant et arrière, et des premier et second plis qui s'étendent longitudinalement dans chacune des marges latérales, **caractérisé en ce qu'**au moins une portion de chacune des oreilles s'étend transversalement au-delà d'au moins une portion du premier pli qui s'étend longitudinalement dans chacune des marges latérales, au moins une portion de chacune des oreilles s'étendant transversalement au-delà de tous les plis qui s'étendent longitudinalement dans les marges latérales.

6. Procédé ou article absorbant jetable plié selon l'une quelconque des revendications précédentes, dans lequel le premier pli qui s'étend longitudinalement est un repli à 180° ou une couture.

7. Procédé ou article absorbant jetable plié selon l'une quelconque des revendications précédentes, dans lequel la portion de la paire d'oreilles inclut des moyens indicateurs pour signaler l'emplacement des oreilles.

8. Procédé ou article absorbant jetable plié selon la revendication 7, dans lequel les moyens indicateurs comprennent une couleur ou un gaufrage.

9. Procédé ou article absorbant jetable plié selon l'une quelconque des revendications précédentes, dans lequel au moins une portion (87) de chacune des oreilles s'étend transversalement au-delà, sur au moins 3 mm, et de préférence au moins 10 mm, du pli qui s'étend longitudinalement.

10. Procédé ou article absorbant jetable plié selon l'une quelconque des revendications précédentes, dans lequel la portion de l'oreille contient un bord distal (94) de l'oreille.

11. Procédé ou article absorbant jetable plié selon la revendication 10, dans lequel la paire d'oreilles (89) est liée à l'article absorbant (30) dans la région arrière.

12. Procédé ou article absorbant jetable plié selon l'une quelconque des revendications précédentes, dans lequel les portions de chacune des oreilles sont situées sensiblement symétriquement par rapport à la ligne centrale longitudinale.

13. Paquet d'articles absorbants jetables pliés comprenant un contenant et au moins un article absorbant jetable plié selon la revendication 5, ou au moins un article absorbant jetable plié selon le procédé de la revendication 1.

14. Article absorbant jetable plié selon la revendication 5, l'article absorbant jetable incluant au moins des premier, second et troisième plis qui s'étendent longitudinalement dans chacune des marges latérales et un pli qui s'étend transversalement, dans lequel au moins une portion de chacune des oreilles s'étend au-delà des premier, second et troisième plis qui s'étendent longitudinalement dans chacune des marges latérales, les portions des oreilles étant colorées pour signaler l'emplacement des portions, les portions s'étendant sur au moins 3 mm au-delà des plis, les portions contenant un bord distal de l'oreille, les portions étant situées sensiblement symétriquement par rapport à la ligne centrale longitudinale, et le pli qui s'étend transversalement étant à l'écart et sensiblement équidistant des bords d'extrémité transversaux opposés.

15. Article absorbant jetable plié selon la revendication 5, dans lequel la paire d'oreilles est associée à la région arrière, l'article absorbant jetable incluant au moins un pli qui s'étend transversalement, le premier pli qui s'étend longitudinalement ayant une largeur dans la région avant et une largeur dans la région arrière, la largeur dans la région avant étant plus étroite que la largeur dans la région arrière, les oreilles étant situées à l'intérieur par rapport au premier pli qui s'étend longitudinalement dans la région arrière, au moins une portion de chacune des oreilles s'étendant au-delà du premier pli qui s'étend longitudinalement dans chacune des marges latérales dans la région avant, les portions des oreilles étant colorées pour signaler l'emplacement des portions, les portions s'étendant sur au moins 3 mm au-delà des plis dans la région avant, les portions contenant un bord distal de l'oreille, les portions étant situées sensiblement symétriquement par rapport à la ligne centrale longitudinale, et le pli qui s'étend transversalement étant à l'écart et sensiblement équidistant des bords d'extrémité transversaux opposés.

16. Procédé ou article absorbant plié selon l'une quelconque des revendications précédentes, l'article comprenant des bords latéraux qui s'étendent longitudinalement (46), transversalement opposés, une feuille dossier (38), une feuille supérieure (40) et un noyau absorbant (42) situé entre la feuille dossier et la feuille supérieure, le noyau absorbant (42) ayant des bords latéraux terminaux qui s'étendent longitudinalement (60), transversalement opposés, lesdits premiers plis qui s'étendent longitudinalement (65) étant formés en pliant les premiers bords latéraux longitudinaux (46) de l'article, vers l'intérieur, en direction d'une surface supérieure (70) de départ de l'article, chaque premier pli qui s'étend longitudinalement étant formé à l'intérieur par rapport au positionnement d'origine du bord latéral longitudinal respectif (46) et soit sur le, soit à l'extérieur par rapport au, bord latéral longitudinal terminal (60) respectif du noyau absorbant (42), et les seconds plis qui s'étendent longitudinalement (67) étant formés en pliant les bords latéraux longitudinaux (46) de l'article, vers l'extérieur, à l'écart de la ligne centrale longitudinale.

17. Procédé ou article absorbant plié selon l'une quelconque des revendications précédentes, dans lequel l'article comprend, en outre, des attaches (82) pour fixer l'article absorbant autour de la taille du porteur.

18. Procédé ou article, selon l'une quelconque des revendications précédentes, dans lequel l'article absorbant jetable (30) est une couche-culotte.

19. Procédé ou article selon l'une quelconque des revendications précédentes, dans lequel, après pliage, le premier pli qui s'étend longitudinalement (65) est à l'extérieur par rapport au second pli qui s'étend longitudinalement (67).
